# EUROPÄISCHE PATENTSCHRIFT

(11) **EP 2 224 871 B1**
(45) Veröffentlichungstag und Bekanntmachung des Hinweises auf die Patenterteilung: **02.05.2012**
(21) Anmeldenummer: 08864217.8
(22) Anmeldetag: 11.12.2008
(51) Int. Cl.: A61B 18/14, A61B 18/12

(54) **CHIRURGIEGERÄTESTECKERSYSTEM**
PLUG SYSTEM FOR SURGICAL DEVICES
SYSTÈME CONNECTEUR D'APPAREIL CHIRURGICAL

(30) Priorität: 20.12.2007 DE 102007061483
(43) Veröffentlichungstag der Anmeldung: 08.09.2010
(73) Patentinhaber: ERBE Elektromedizin GmbH, 72072 Tübingen (DE)
(72) Erfinder: BELLER, Jürgen, 72810 Gomaringen (DE); EISELE, Florian, 79108 Freiburg (DE)
(74) Vertreter: Bohnenberger, Johannes
(86) Internationale Anmeldenummer: PCT/EP2008/010555
(87) Internationale Veröffentlichungsnummer: WO 2009/080221

(56) Entgegenhaltungen:
- EP-A- 0 819 409
- DE-A1- 10 253 819
- DE-A1-102006 022 606
- US-A- 5 573 533
- US-A- 6 113 596
- US-A1- 2004 054 365
- US-A1- 2005 192 609
- US-A1- 2006 200 120
- US-B1- 6 402 743

## Beschreibung

Die Erfindung betrifft ein Chirurgiegerätesteckersystem.

In der Chirurgie werden Elektrochirurgiegeräte verwendet, an welche elektrochirurgische Instrumente angeschlossen werden können. Dabei ist es üblich, an dasselbe Elektrochirurgiegerät verschiedene Instrumente anzuschließen bzw. umgekehrt dasselbe Instrument an verschiedene Elektrochirurgiegeräte anzuschließen. Die elektrochirurgischen Instrumente können im allgemeinen Fall an das Elektrochirurgiegerät über elektrische Leitungen gleichzeitig oder nacheinander angeschlossen werden. Das Elektrochirurgiegerät erzeugt elektrische Spannungen bzw. Ströme zum monopolaren, bipolaren oder auch höherpolaren Schneiden und/oder Koagulieren biologischen Gewebes mittels der elektrochirurgischen Instrumente.

Üblicherweise werden für Verbindungsleitungen, die zwischen dem Elektrochirurgiegerät und dem Instrument angeordnet sind, verschiedene Chirurgiegerätesteckersysteme verwendet. Es hat sich dabei in der Vergangenheit eine Vielzahl solcher Chirurgiegerätesteckersysteme im Markt etabliert.

Um die Vielzahl der am Markt befindlichen Elektrochirurgieinstrumente an dasselbe Elektrochirurgiegerät anschließen zu können, sind je nach Aufbau des Elektrochirurgiegeräts verschiedene Anschlussbuchsen ausgebildet. Um z. B. ein Instrument mit einer monopolaren Anschlussleitung an ein Elektrochirurgiegerät anzuschließen, wird eine monopolare Anschlussbuchse verwendet, soll aber ein Instrument mit einer bipolaren Anschlussleitung an einem Elektrochirurgiegerät angeschlossen werden, muss eine bipolare Anschlussbuchse am Elektrochirurgiegerät vorhanden sein. Dies führt zu einer Vielzahl von Anschlussbuchsen am Elektrochirurgiegerät mit der Folge, dass oftmals insbesondere in der hektischen Atmosphäre eines Operationssaals Elektrochirurgieinstrumente in falsche Buchsen eingeführt werden. Dies wiederum hat zur Folge, dass eine gewisse Zeit verstreicht bis das fälschliche Einstecken bemerkt wird und das Instrument in die richtige Anschlussbuchse eingesteckt wird. Es ist sogar denkbar, dass in Folge eines fälschlichen Einsteckens das Elektrochirurgieinstrument beschädigt wird, was schlimme Folgen für den zu operierenden Patenten haben kann.

Aus der US 2004/05436 A1, welches den Gegenstand der Präambel von Anspruch 1 offenbart, ist ein elektrochirurgisches System mit einem elektrochirurgischen Generator und einem elektrochirurgischen Instrument zum Schneiden und/oder Koagulieren von Gewebe bekannt. Das elektrochirurgische Instrument weist ein Identifikationselement auf, das mindestens die Anzahl der Elektroden des Instrumentes repräsentiert. Das Identifikationselement kann beispielsweise ein Widerstand oder eine Kapazität sein. Aus der US 6 113 596 A ist ein elektrochirurgisches System mit einem entsprechenden Instrument bekannt, wobei ein Kabel vorgesehen ist, das entweder im monopolaren oder bipolaren Modus zum Einsatz kommen kann. Das Kabel kann dabei eine Steuerung umfassen, die eine Buchse entsprechend schaltet, so dass das elektrochirurgische Instrument entweder mit dem "monopolaren" oder "bipolaren" Anschluss eines HF-Generators verbunden ist. Ein Relais schließt bipolare Kontakte an, wenn durch die Steuerung ein bipolarer Strom gemessen wird. Sobald kein biopolarer Strom gemessen wird, werden entsprechend monopolare Kontakte angeschlossen. Die DE 10 2006 022 606 A1 offenbart ein Adapterkabel mit einem programmierbaren Speicher. Die US 2005/0192609 A1 betrifft ein elektromechanisches Chirurgiegerät mit einer Fernbedienung, die ein Gehäuse und ein Paneel mit einem Display umfasst. Die US 5 573 533 A offenbart eine Kathetervorrichtung, bei der festgestellt werden kann, ob ein Katheter bereits benutzt wurde oder nicht.

Der Erfindung liegt die Aufgabe zugrunde, ein vereinfachtes Chirurgiegerätesteckersystem aufzuzeigen, bei dem die Sicherheit erhöht ist.

Diese Aufgabe wird durch ein Chirurgiegerätesteckersystem nach Patentanspruch 1 gelöst.

Insbesondere wird die Aufgabe durch ein Chirurgiegerätesteckersystem gelöst, umfassend eine Vielzahl von Steckbuchsen, in welche Verbindungsstecker von chirurgischen Instrumenten einsteckbar sind, eine Schaltmatrix, die eine Vielzahl von Schaltern umfasst, zum Verbinden mindestens einer der Steckbuchsen mit mindestens zwei verschiedenen Aus- oder Eingängen eines Elektrochirurgiegerätes und eine Steuerung mit einer Vorwahleinrichtung zum Steuern der Schaltmatrix derart, dass die Schaltmatrix entsprechend in die Vorwahleinrichtung eingegebener Daten vorbestimmte Steckbuchsen mit vorbestimmten Ein- oder Ausgängen verbindet. Die Steuerung umfasst ein Speicherelement zum Speichern einer Basiskonfiguration einer Verbindung zwischen Steckbuchsen und Ein-/Ausgängen des Elektrochirurgiegeräts, wobei die Basiskonfiguration eingestellt ist, wenn keine Dateneingabe in der Vorwahleinrichtung erfolgt, wobei in der Basiskonfiguration alle Schalter der Schaltmatrix geöffnet sein. Dadurch wird sichergestellt, dass keine falschen Verbindungen bei einem Einführen des Verbindungssteckers des elektrochirurgischen Instruments hergestellt werden.

Ein wesentlicher Punkt der Erfindung liegt darin, dass das Chirurgiegerätesteckersystem für eine Vielzahl verschiedener elektrochirurgischer Instrumente bzw. Elektrochirurgiegeräte verwendet wird, wobei eine Schaltmatrix nach der Eingabe einer Codierung, die das anzuschließende elektrochirurgische Instrument identifiziert, derart konfiguriert wird, dass vorbestimmte Steckbuchsen mit vorbestimmten Ein- oder Ausgängen des Elektrochirurgiegerätes verbunden werden. Dadurch wird die Anzahl der notwendigen Steckbuchsen verringert und die Sicherheit erhöht.

Bei einer ersten Ausführungsform der Erfindung umfasst die Vorwahleinrichtung eine lösbare Verbindungseinrichtung, über welche eine Verbindung zum elektrochirurgischen Instrument zur Eingabe der Daten herstellbar ist. Eine solche Verbindungseinrichtung erleichtert das Übermitteln der Daten.

Vorzugsweise umfasst die Verbindungseinrichtung eine Decodierbuchse, in welche ein Codierstecker des chirurgischen Instruments einsteckbar ist, der getrennt von den Verbindungssteckern ist. Die von den Verbindungssteckern getrennte Ausbildung des Codiersteckers ermöglicht es, konventionelle Verbindungsstecker weiterhin zu verwenden.

Vorzugsweise ist das Elektrochirurgiegerät ein Hochfrequenz(HF)-Chirurgiegerät. Insbesondere in der HF-Chirurgie ist die Anzahl der verschiedenen Instrumente hoch und das Chirurgiegerätesteckersystem daher besonders nützlich.

In einer weiteren Alternative speichert das Speicherelement eine jeweils zuletzt bestimmte Verbindungskonfiguration. Dies ist besonders dann vorteilhaft, wenn am selben Elektrochirurgiegerät ein bestimmtes Elektrochirurgieinstrument besonders häufig verwendet wird, so dass auch bei einem Defekt des Codiersteckers, eine für das bestimmte Instrument geeignete Verbindungskonfiguration durch das Speicherelement zur Verfügung gestellt wird.

Vorzugsweise sind die Daten manuell in die Vorwahleinrichtung des Chirurgiegerätesteckersystems eingebbar. Dies ist insbesondere dann von Vorteil, wenn an einem bestimmten Elektrochirurgieinstrument kein Codierstecker vorgesehen ist oder ein solcher ausfällt. In diesem Fall können dann die Daten, die beispielsweise auf dem Verbindungsstecker aufgedruckt sind, abgelesen werden und manuell in die Vorwahleinrichtung eingegeben werden, so dass das elektrochirurgische Instrument verwendet werden kann.

Weitere Ausführungsformen der Erfindung ergeben sich aus den Unteransprüchen.

Nachfolgend wird die Erfindung auch hinsichtlich weiterer Merkmale und Vorteile anhand eines Ausführungsbeispiels beschrieben, das anhand der Abbildungen näher erläutert wird.

Hierbei zeigen:
- Fig. 1: eine erste Ausführungsform der Erfindung;
- Fig. 2: eine Buchsen- und Steckeranordnung der Ausführungsform nach Fig. 1.

In der nachfolgenden Beschreibung werden für gleiche oder gleich wirkende Teile dieselben Bezugsziffern verwendet.

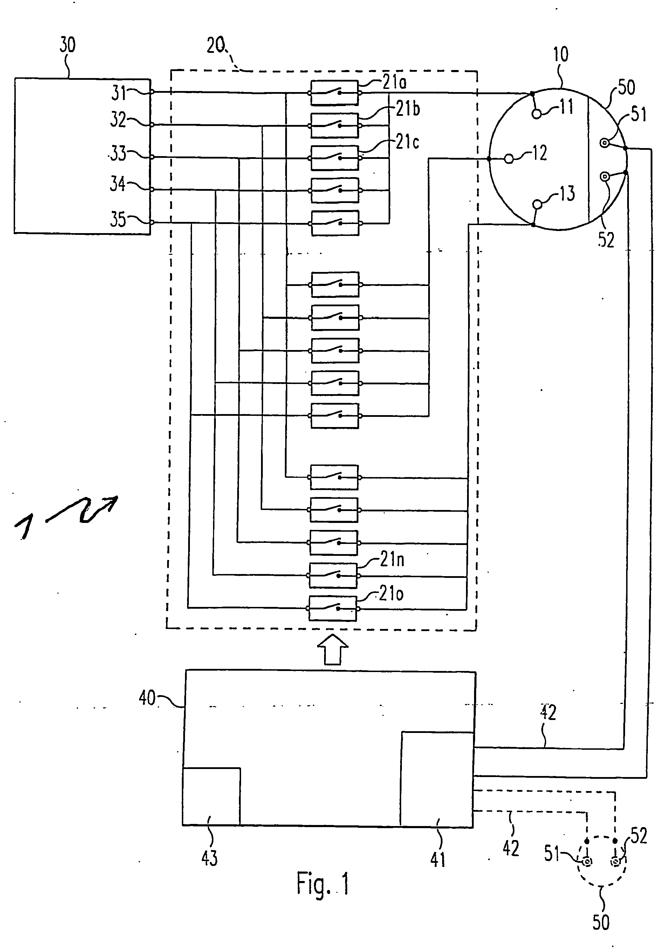

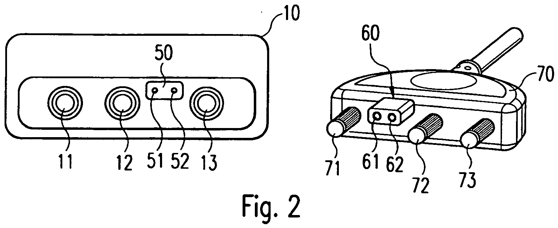

Das in Fig. 1 dargestellte Chirurgiegerätesteckersystem 1 umfasst eine Steckbuchse 10 mit Steckkontakten 11-13, die über eine Schaltmatrix 20 mit Ein-/Ausgängen 31-35 eines Elektrochirurgiegerätes 30 verbunden sind und einer Steuerung 40, die Daten eines Codiersteckers 60 (siehe Fig. 2), die über eine Decodierbuchse 50 in eine Vorwahleinrichtung 41 eingegeben werden, analysiert und daraufhin die Schaltmatrix 20 derart steuert, dass Schalter 21a-21o der Schaltmatrix 20 geschlossen werden und dadurch eine elektrische Verbindung zwischen den Steckkontakten 11-13 der Steckbuchse 10 und den Ein-/Ausgängen 31-35 des Elektrochirurgiegerätes 30 realisiert wird.

In der Ausführungsform nach Fig. 1 und 2 weist das Chirurgiegerätesteckersystem 1 eine Steckbuchse 10 mit drei Steckkontakten 11-13 auf, die mit fünf verschiedenen Ein-/Ausgängen 31-35 des Elektrochirurgiegerätes 30 verbunden werden können. Im Allgemeinfall ist es natürlich denkbar, dass eine beliebige Anzahl von Steckbuchsen 10 die jeweils eine beliebige Anzahl von Steckkontakten 11-13 aufweisen, mit den Ein-/Ausgängen 31-35 verbunden wird.

In Fig. 2 ist eine mögliche Anordnung von Steckbuchse 10 und Decodierbuchse 50 auf der einen Seite und Verbindungsstecker 70 und Codierstecker 60 auf der anderen Seite dargestellt. Der Codierstecker 60 ist hierbei am Verbindungsstecker 70 derartig angebracht, dass bei einem Einführen von Kontaktstiften 71-73 des Verbindungssteckers 70 in die Steckkontakte 11-13 der Steckbuchse 10 automatisch auch Kontaktstifte 51, 52 der Decodierbuchse 50, die innerhalb der Steckbuchse 10 angebracht ist, in Steckkontakte 61, 62 des Codiersteckers 60 eingeführt werden.

Denkbar in einer alternativen Ausführungsform ist es auch, dass der Codierstecker 60 räumlich getrennt vom Verbindungsstecker 20 am elektrochirurgischen Instrument angebracht ist. Ebenso - wie in Fig. 1 durch den gestrichelt gezeichneten Bereich der Figur illustriert - können Steckbuchse 10 und Decodierbuchse 50 getrennt vorliegen. In einer solchen Ausführungsform werden in einem ersten Schritt Kontaktstifte 51, 52 der Decodierbuchse 50 in Steckkontakte 61, 62 des Codiersteckers 60 eingeführt, woraufhin die Steuerung 40 die Schalter 21a-21o der Schaltmatrix 20 entsprechend einstellt, woraufhin in einem zweiten Schritt die Kontaktstifte 71-73 des Verbindungsstecker 70 in die Steckbuchse 10 eingeführt werden. Bei einer solchen Ausführungsform können konventionelle Verbindungsstecker 70 verwendet werden.

Die Steuerung 40 umfasst ein Speicherelement 43 zur Speicherung mindestens einer Konfiguration der Schaltungsmatrix 20.

Die Übertragung der Daten vom elektrochirurgischen Instrument an die Steuerung 40 erfolgt im Ausführungsbeispiel nach Fig. 1 und 2 über eine drahtgebundene Verbindungseinrichtung 42.

Die Übertragung der Daten vom elektrochirurgischen Instrument an die Steuerung 40 kann auch drahtlos erfolgen. Bei einer solchen drahtlosen Übertragung der Daten, kann eine dem Codierstecker 60 in seiner Funktion entsprechende Codiereinrichtung (nicht in Figuren gezeigt) an einer beliebigen Stelle des elektrochirurgischen Instruments angebracht sein, sowie eine Decodiereinrichtung, die in ihrer Funktion der Decodierbuchse 50 entspricht, an einer beliebigen Stelle der Steuerung 40 bzw. des Elektrochirurgiegerätes 30.

Aus obigem geht hervor, dass die vorigen Erfindungen nicht nur ein Chirurgiegerätesteckersystem 1 betrifft, sondern auch ein Verfahren zur Einstellung eines Chirurgiegerätesteckersystems 1.

Besonders vorteilhaft ist die Verwendung des Chirurgiegerätesteckersystems in der HF-Chirurgie, da hier besonders viele verschiedene elektrochirurgische Instrumente zum Einsatz kommen.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 1: Chirurgiegerätesteckersystem
- 10: Steckbuchse
- 11-13: Steckkontakt
- 20: Schaltmatrix
- 21a-21o: Schalter
- 30: Elektrochirurgiegerät
- 31-35: Ein-/Ausgang
- 40: Steuerung
- 41: Vorwahleinrichtung
- 42: Verbindungseinrichtung
- 43: Speicherelement
- 50: Decodierbuchse
- 51, 52: Kontaktstift
- 60: Codierstecker
- 61, 62: Steckkontakt
- 70: Verbindungsstecker
- 71-73: Kontaktstift

## Patentansprüche

1. Chirurgiegerätesteckersystem (1), umfassend
mindestens eine Steckbuchse (10) mit einer Vielzahl von Steckkontakten (11-13), in welche Kontaktstifte (71-73) von Verbindungssteckern (70) von elektrochirurgischen Instrumenten einsteckbar sind,
eine Schaltmatrix (20), die eine Vielzahl von Schaltern (21a-21o) umfasst, zum Verbinden mindestens einer der Steckbuchsen (10) mit mindestens zwei verschiedenen Ein- oder Ausgängen (31-35) eines Elektrochirurgiegerätes (30) und eine Steuerung (40) mit einer Vorwahleinrichtung (41) zum Steuern der Schaltmatrix (20) derart, dass die Schaltmatrix (20) entsprechend in die Vorwahleinrichtung (41) eingegebener Daten vorbestimmte Steckbuchsen (10) mit vorbestimmten Ein- oder Ausgängen (31-35) verbindet, daduch gekennzeichnet, dass die Steuerung (40) ein Speicherelement (43) zum Speichern einer Basiskonfiguration einer Verbindung zwischen Steckbuchsen (10) und Ein-/Ausgängen (31-35) des Elektrochirurgiegeräts (30) umfasst, wobei die Basiskonfiguration eingestellt ist, wenn keine Dateneingabe in die Vorwahleinrichtung erfolgt, wobei in der Basiskonfiguration alle Schalter geöffnet sind.

2. Chirurgiegerätesteckersystem (1) nach Anspruch 1,
**dadurch gekennzeichnet, dass**
die Vorwahleinrichtung (41) eine lösbare Verbindungseinrichtung (42) umfasst, über welche eine Verbindung zum elektrochirurgischen Instrument zur Eingabe der Daten herstellbar ist.

3. Chirurgiegerätesteckersystem (1) nach Anspruch 1 oder 2, insbesondere nach Anspruch 2,
**dadurch gekennzeichnet, dass**
die Verbindungseinrichtung (42) mindestens eine elektrische Steckbuchse (50) umfasst.

4. Chirurgiegerätesteckersystem (1) nach einem der vorhergehenden Ansprüche, insbesondere nach Anspruch 3,
**dadurch gekennzeichnet, dass** in die elektrische Steckbuchse (50) ein Codierstecker (60) des elektrochirurgischen Instruments einsteckbar ist, der getrennt von den Verbindungssteckern (70) ist.

5. Chirurgiegerätesteckersystem (1) nach einem der vorhergehenden Ansprüche,
**dadurch gekennzeichnet, dass** das Elektrochirurgiegerät (30) ein HF-Chirurgiegerät ist.

6. Chirurgiegerätesteckersystem (1) nach einem der Ansprüche 2-5,
**dadurch gekennzeichnet, dass** das Speicherelement (43) eine jeweils zuletzt bestimmte Verbindungskonfiguration speichert.

7. Chirurgiegerätesteckersystem (1) nach einem der vorhergehenden Ansprüche,
**dadurch gekennzeichnet, dass** die Daten manuell in die Vorwahleinrichtung (41) eingebbar sind.

## Claims

1. Surgical device plug system (1), comprising
at least one socket (10) with a multiplicity of plug-in contacts (11-13), into which contact pins (71-73) of connection plugs (70) of electrosurgical instruments can be plugged,
a switching matrix (20), which comprises a multiplicity of switches (21a-21o), for connecting at least one of the sockets (10) to at least two different inputs or outputs (31-35) of an electrosurgical device (30) and
a control (40) with a selector apparatus (41) for controlling the switching matrix (20) such that the switching matrix (20), in accordance with data entered into the selector apparatus (41), connects predetermined sockets (10) to predetermined inputs or outputs (31-35), **characterized in that** the control (40) comprises a storage element (43) for storing a base configuration of a connection between sockets (10) and inputs/outputs (31-35) of the electrosurgical device (30), the base configuration being set if no data is entered into the selector apparatus (41), with all switches being open in the base configuration.

2. Surgical device plug system (1) according to Claim 1,
**characterized in that**
the selector apparatus (41) comprises a detachable connection apparatus (42), by means of which a connection to the electrosurgical instrument can be established for entering the data.

3. Surgical device plug system (1) according to Claim 1 or 2, more particularly according to Claim 2,
**characterized in that**
the connection apparatus (42) comprises at least one electrical socket (50).

4. Surgical device plug system (1) according to one of the preceding claims, more particularly according to Claim 3,
**characterized in that**
a coding plug (60), which is separate from the connection plugs (70), of the electrosurgical instrument can be plugged into the electrical socket (50).

5. Surgical device plug system (1) according to one of the preceding claims,
**characterized in that**
the electrosurgical device (30) is an RF surgical device.

6. Surgical device plug system (1) according to one of Claims 2-5,
**characterized in that**
the storage element (43) stores a respectively last-determined connection configuration.

7. Surgical device plug system (1) according to one of the preceding claims,
**characterized in that**
the data can be entered manually into the selector apparatus (41).

## Revendications

1. Système de connecteur pour appareil chirurgical (1) comprenant au moins une douille de connexion (10) ayant une pluralité de contacts d'enfichage (11-13) dans lesquels peuvent être insérées des broches de contact (71-73) de connecteurs (70) d'instruments électro-chirurgicaux,
une matrice de commutation (20) qui comprend une pluralité de commutateurs (21a-21o) pour le raccordement d'au moins l'une des douilles de connexion (10) à au moins deux entrées ou sorties différentes (31-35) d'un appareil électro-chirurgical (30) et une unité de commande (40) comportant un dispositif de présélection (41) destiné à commander la matrice de commutation (20) de telle manière que la matrice de commutation (20) connecte, conformément à des données fournies en entrée dans le dispositif de présélection (41), des douilles de connexion (10) prédéterminées à des entrées ou des sorties (31-35) prédéterminées, **caractérisé en ce que** l'unité de commande (40) comprend un élément de mémoire (43) destiné à stocker une configuration de base d'une connexion entre des douilles de connexion (10) et des entrées/sorties (31-35) de l'appareil électro-chirurgical (30), dans lequel la configuration de base est établie lorsqu'aucune entrée de données n'est effectuée dans le dispositif de présélection, les commutateurs étant tous ouverts dans ladite configuration de base.

2. Système de connecteur pour appareil chirurgical (1) selon la revendication 1,
**caractérisé en ce que**
le dispositif de présélection (41) comprend un dispositif de connexion amovible (42) par l'intermédiaire duquel une connexion à un instrument électro-chirurgical est effectuée pour l'entrée des données.

3. Système de connecteur pour appareil chirurgical (1) selon la revendication 1 ou 2, notamment selon la revendication 2,
**caractérisé en ce que**
le dispositif de connexion (42) comprend au moins une douille de connexion électrique (50).

4. Système de connecteur pour appareil chirurgical (1) selon l'une des revendications précédentes, notamment selon la revendication 3,
**caractérisé en ce**
**qu'**un connecteur de codage (60) de l'instrument électro-chirurgical qui est séparé des connecteurs (70) peut être enfiché dans la douille de connexion électrique (50).

5. Système de connecteur pour appareil chirurgical (1) selon l'une des revendications précédentes,
**caractérisé en ce que**
l'appareil électro-chirurgical (30) est un appareil chirurgical HF.

6. Système de connecteur pour appareil chirurgical (1) selon l'une des revendications 2-5,
**caractérisé en ce que**
l'élément de mémoire (43) stocke une configuration de connexion respective déterminée en dernier.

7. Système de connecteur pour appareil chirurgical (1) selon l'une des revendications précédentes,
**caractérisé en ce que**
les données sont fournies en entrée manuellement dans le dispositif de présélection (41).
